# EUROPEAN PATENT SPECIFICATION

(11) **EP 3 677 336 B1**
(45) Date of publication and mention of the grant of the patent: **28.04.2021**
(21) Application number: 20153233.0
(22) Date of filing: 03.09.2008
(51) Int. Cl.: B01L 3/00, C12Q 1/42, G01N 30/88

(54) **MICROFLUIDIC METHOD AND SYSTEM FOR ENZYME INHIBITION ACTIVITY SCREENING**
MIKROFLUIDISCHES VERFAHREN UND SYSTEM ZUM SCREENING VON ENZYMHEMMUNGSAKTIVITÄT
PROCÉDÉ MICROFLUIDIQUE ET SYSTÈME DE CRIBLAGE D'ACTIVITÉ POUR L'INHIBITION D'ENZYMES

(30) Priority: 06.09.2007 US 899599
(43) Date of publication of application: 08.07.2020
(62) Divisional of application: 08828969.9
(73) Proprietor: Caliper Life Sciences Inc., Mount View, California 94043 (US)
(72) Inventor: DUNNE, Jude, A., Menlo Park, CA 94025 (US); FARINAS, Javier, A., Los Altos, CA 94024 (US); KAZAKOVA, Irina, G., Los Gatos, CA 95032 (US); HUANG, Esther, Mountain View, CA 94043 (US); GERBER, Raphaele, Foster City, CA 94404 (US); HACKER, Coleen, Cupertino, CA 95014 (US); DETTLOFF, Roger, Emerald Hills, CA 94062 (US); TRINH, Thi Ngoc Vy, Cupertino, CA 95014 (US)
(74) Representative: Mewburn Ellis LLP

(56) References cited:
- WO-A-00/72016
- WO-A-00/75167
- WO-A-96/04547
- US-A1- 2005 221 385

## Description

### FIELD OF THE INVENTION

The present invention relates to kits, systems, and methods for determining the level of enzyme activity in the presence of a compound, and more particularly to kits, systems, and methods for identifying drugs that modulate kinase activity across an array of kinases.

### BACKGROUND OF THE INVENTION

Kinases are enzymes that catalyze the transfer of a phosphate group from ATP (adenosine triphosphate) or another nucleoside triphosphate to a substrate. For example, hexokinase catalyzes the phosphoryl transfer from ATP to glucose to produce glucose-6-phosphate as an initial step in glycolysis. Other kinases involved in glycolysis include phosphofructokinase, phosphoglycerate kinase, and pyruvate kinase. Kinases are also involved, e.g., in protein phosphorylation by transferring the terminal phosphate from ATP to a side chain of an amino acid residue of a protein. In eukaryotic cells, protein phosphorylation serves various functions including, e.g., the phosphorylation of cell-surface receptors to produce intracellular effects, the regulation of the cell cycle, the protection of cells from toxic changes in metabolites, or the like.

Protein kinases comprise a large family of enzymes with over 500 different forms identified to date in the human genome. Protein substrates for these enzymes may include up to one-third of all cellular proteins. An understanding of the protein kinase enzymes and their targets is crucial to understanding cellular regulation and cellular pathology.

Protein kinases play very important roles in many cell functions, including, but not limited to, cellular metabolism, signal transduction, transcriptional regulation, cell motility, cell division, cellular signaling processes, cellular proliferation, cellular differentiation, apoptosis, and secretion. These processes are mediated by phosphorylation or dephosphorylation of enzymes, protein substrates, transcription factors, hormone or growth factor receptors, and other cellular proteins. Protein phosphorylation is a regulatory mechanism used by cells to selectively modify proteins carrying regulatory signals from outside the cell to the nucleus.

Protein kinases are also involved in mediating the response to naturally occurring toxins and pathogens, which alter the phosphorylation states of proteins. Additionally, protein kinases are related to many epidemiologically relevant oncogenes and tumor suppressor genes. In fact, protein kinases are implicated in several hundred human diseases. Examples include neurodegenerative diseases such as amyotrophic lateral sclerosis and Alzheimer's disease. Consequently, protein kinases are often validated drug targets since human diseases are frequently linked to dysregulation of cellular signaling pathways.

With phosphorylation involved in so many cell functions and diseases, identifying compounds that affect protein kinase activity is tremendously important, not only to provide drug candidates that are active in treating disease, but also to ensure that these drug candidates act without causing adverse effects on other cellular functions. Thus, there is a need for high-throughput screening assays to identify activators and inhibitors of kinases as well as kits for carrying out such assays. Assay devices are known from US 2005/221385 A1, WO 00/72016 A, WO 96/04547 A and WO 00/75167 A.

### SUMMARY OF THE INVENTION

In a first aspect there is provided a kit for screening a compound for enzyme inhibition activity using a microfluidic device 110, the microfluidic device having at least one microchannel 102 and a capillary element 112, the capillary element operably connected to and in fluid communication with the microchannel, the kit comprising: a first multiwell plate 150 having a plurality of enzymes disposed within a first plurality of wells; a second multiwell plate 160 having a plurality of enzyme substrates disposed within a second plurality of wells, each one of the second plurality of wells corresponding to one of the first plurality of wells; a phosphate source 162 disposed within each well of the second plate, the phosphate source to be disposed in each of the well at a predetermined phosphate source concentration; a cofactor 164 disposed within each well of the second plate, the cofactor disposed in each well at a predetermined cofactor concentration; wherein the enzyme within each well of the first plate is selected to react with the enzyme substrate, the phosphate source, and the cofactor in the corresponding well of the second plate when the compound is not present; and an instruction set stored on a computer readable medium, the instruction set comprising instructions for controlling dipping of capillary elements into the first plurality of wells; wherein the microfluidic device has a first reservoir at one end of the microchannel and a second reservoir at an opposite terminus of the microchannel, the first reservoir having a depth greater than the depth of the second reservoir, the first reservoir having a larger volume relative to the volume or flow rate of the at least one microchannel such that a sample is flowable into the at least one microchannel of the microfluidic device via the capillary element of the microfluidic device by a hydrostatic pressure differential created by the depth differential between the first reservoir and the second reservoir.

Optionally, the first plurality of wells of the first multiwell plate are disposed evenly upon the plate in 24 columns 250, each column configured for including a different enzyme disposed within the wells of the column.

Optionally, the order in which the enzymes are disposed on the first plate is based on similarity of electrophoretic mobility.

Optionally, the enzymes are disposed on the first plate from column 1 to column 24 is MAPKAPK2, AurA, PKCζ RSK1, MAPKAPK5, Erk1, PKD2, CK1δ, CHK1, ABL, FYN, LYNa, CHK2, MET, LCK, SRC, GSK3β, Erk2, PKACα, AKT2, INSR, p38a, AKT1, and MSK1.

Optionally, the order in which the enzymes are disposed on the first plate from column 1 to column 24 is PKCβ2, ROCK2, CDK2, MST2, PKG1α, PAK2, IGF1R, FGFR1, MARK1, CAMK26, PIM2, BTK, c-TAK1, DYRK1a, CaMK4, AMPKα1, FLT3, HGK, KDR, Raf-1, P70S6K, IRAK4, SGK, and SYK

Optionally, the kit further comprises a reconstitution buffer 152.

Optionally, the kit further comprises a termination buffer 158.

In a **second aspect** there is a system for screening a compound for enzyme inhibition activity, the system comprising: a kit including a first multiwell plate 150 and a second multiwell plate 160, the first multiwell plate having a plurality of enzymes disposed within a first plurality of wells, the second multiwell plate having a plurality of enzyme substrates disposed within a second plurality of wells, each one of the second plurality of wells corresponding to one of the first plurality of wells, a phosphate source 162 disposed at a predetermined phosphate source concentration within each well of the second multiwell plate, a cofactor 164 disposed at a predetermined cofactor concentration within each well of the second multiwell plate, and wherein the enzyme for being disposed within each well of the first plate is selected to react with the enzyme substrate, the phosphate source, and the cofactor in the corresponding well of the second plate when the compound is not present; a microfluidic device 110, the microfluidic device having at least one microchannel 102 and a capillary element 112, the capillary element operably connected to and in fluid communication with the microchannel;
and an instruction set stored on a computer readable medium, the instruction set comprising instructions for controlling dipping of the capillary element into a respective well of the first plurality of wells; wherein the microfluidic device has a first reservoir at one end of the microchannel and a second reservoir at an opposite terminus of the microchannel, the first reservoir having a depth greater than the depth of the second reservoir, the first reservoir having a large volume relative to the volume or flow rate of the at least one microchannel, such that a sample is flowable into the at least one microchannel of the microfluidic device via the capillary element of the microfluidic device by a hydrostatic pressure differential created by the depth differential between the first reservoir and the second reservoir.

Optionally, the system further comprises a detector 122 operably connected to the microfluidic device and a computer 120.

Optionally, the system further comprises a controller 130 operably connected to the computer 120.

Optionally, the system further comprised a fluid direction system 140 operably connected to the computer 120, the fluid direction system including a pressure source 142 in fluid communication with the microfluidic device.

Optionally, the plurality of enzymes are sealed in the first plurality of wells, and the plurality of enzyme substrates, the phosphate source, and the cofactor are sealed in within the second plurality of wells.

Optionally, at least some of the first plurality of wells are control wells.

Optionally, the plurality of enzymes are sealed in the first plurality of wells, and the plurality of enzyme substrates, the phosphate source, and the cofactor are sealed in the second plurality of wells.

Optionally, at least some of the first plurality of wells are control wells.

The present invention is illustrated by the accompanying drawings of various embodiments and the detailed description given below. The drawings should not be taken to limit the invention to the specific embodiments, but are for explanation and understanding. The detailed description and drawings are merely illustrative of the invention rather than limiting, the scope of the invention being defined by the appended claims and equivalents thereof.

### BRIEF DESCRIPTION OF THE DRAWINGS

**FIG. 1** schematically illustrates a system for screening a compound for enzyme inhibition activity, in accordance with the present invention;
**FIG. 2** illustrates one embodiment of an enzyme plate used in accordance with the present invention;
**FIG. 3** is a table illustrating components and reaction conditions for one embodiment of a screening kit, in accordance with the present invention;
**FIG. 4** is a table illustrating components and reaction conditions for another embodiment of a screening kit, in accordance with the present invention;
**FIG. 5** schematically illustrates a portion of a microfluidic device such as is illustrated in **FIG. 1****,** in accordance with the present invention;
**FIG. 6** illustrates enzyme degradation data for ABL;
**FIG. 7** illustrates the change in conversion per week for a subset of enzymes that was tested for degradation over 22 weeks (error bars = 95% confidence intervals);
**FIG. 8** illustrates selectivity data for staurosporine;
**FIG. 9** illustrates selectivity data for K252A; and
**FIG. 10** illustrates selectivity data SB203580.

The drawings are not to scale.

### DETAILED DESCRIPTION

Before describing the present invention in detail, it is to be understood that this invention is not limited to particular compositions, devices, or systems, which can, of course, vary. It is also to be understood that the terminology used herein is for the purpose of describing particular embodiments only and is not intended to be limiting. Further, unless defined otherwise, all technical and scientific terms used herein have the same meaning as commonly understood by one of ordinary skill in the art to which the invention pertains.

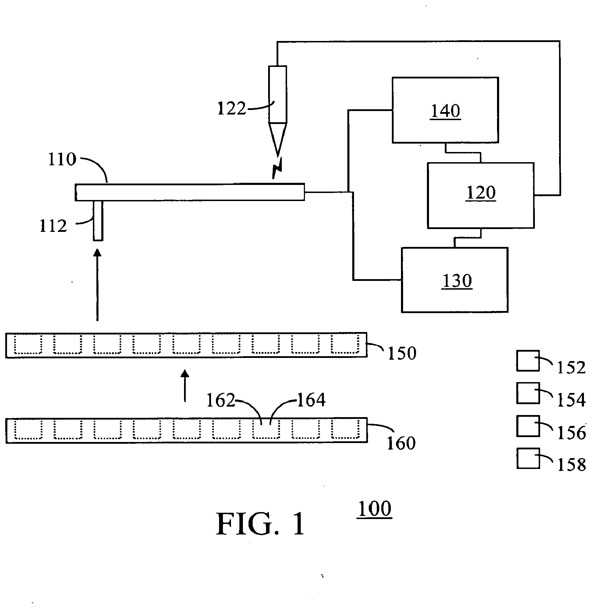

One aspect of the present invention is a kit for screening a compound for enzyme inhibition activity. In one embodiment, the kit includes first and second multiwell plates, i.e., enzyme plate **150** and substrate plate **160,** a reconstitution buffer **152,** DTT (dithiothreitol) **154,** a protease inhibitor **156,** and a termination buffer **158,** all of which components are included in the system illustrated at **100** in **FIG. 1****.** A plurality of enzymes are disposed within the wells of enzyme plate **150,** and a plurality of enzyme substrates, a phosphate source **162,** and a cofactor **164** are disposed within the wells of substrate plate **160.**

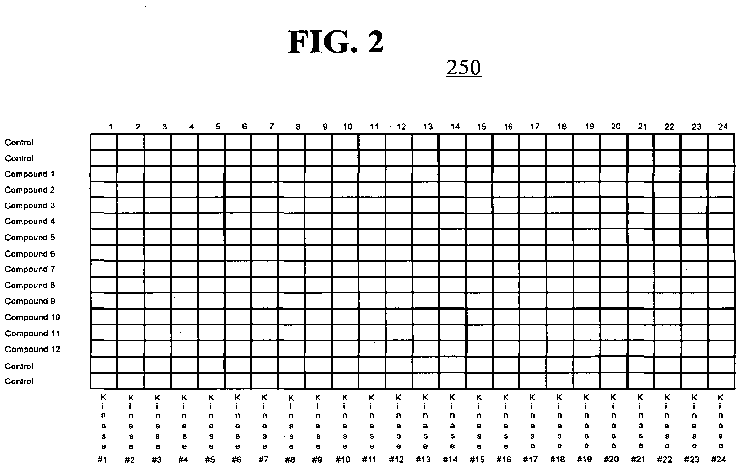

In one embodiment, enzyme plate **150** is an enzyme plate such as is illustrated in **FIG. 2** at **250.** Enzyme plate **250** includes a plurality of enzymes disposed within individual wells of plate **250.** The arrangement of the enzymes on the enzyme plate is determined by the positioning of the corresponding substrates on the substrate plate. Positioning of the substrates is based on factors such as, for example, electrophoretic mobility.

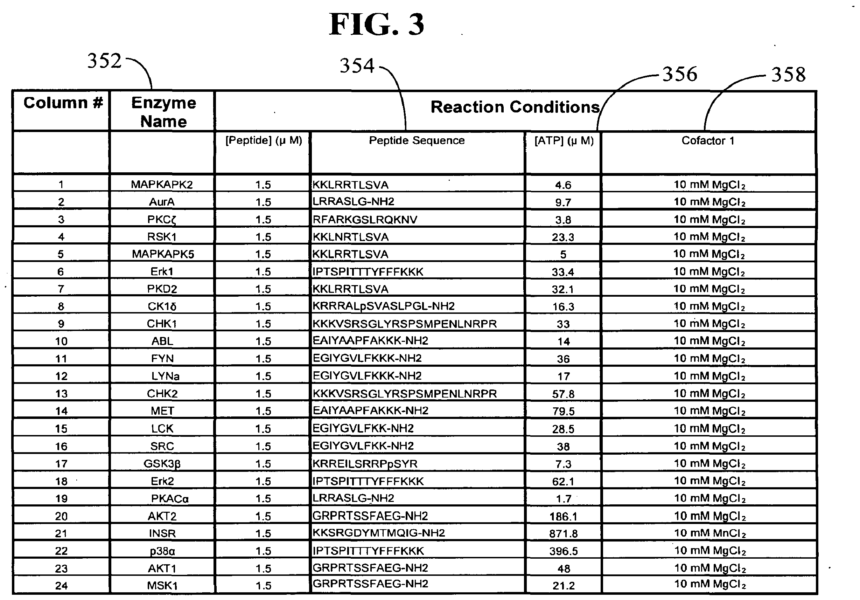

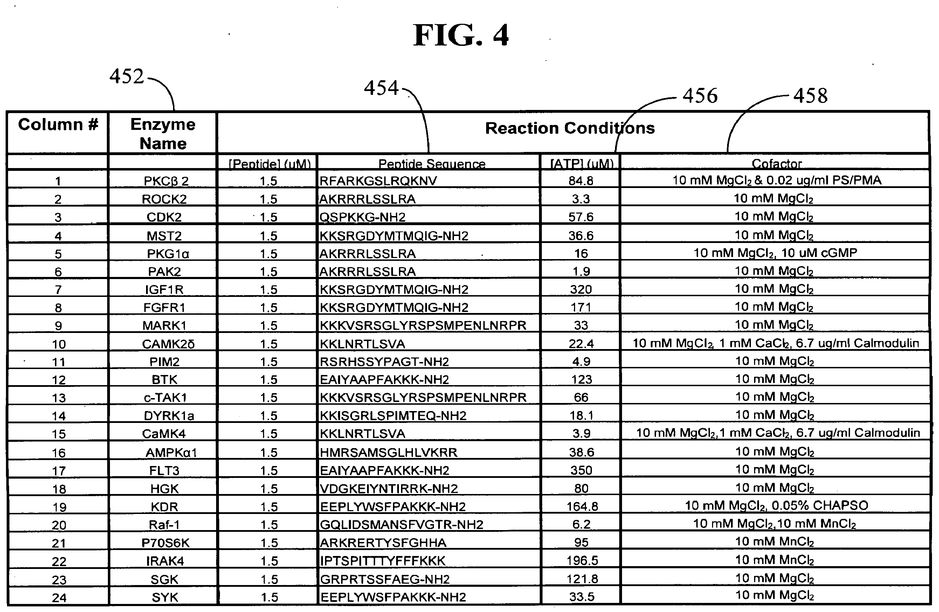

In this first illustrative embodiment, enzyme plate **250** includes a plurality of kinases numbered 1 through 24 disposed in columns 1 through 24 of plate **250.** Kinases 1 through 24 comprise enzymes **352** illustrated in **FIG. 3****.** Enzymes **352** include kinases MAPKAPK2, AurA, PKCζ, RSK1, MAPKAPK5, Erk1, PKD2, CK1δ, CHK1, ABL, FYN, LYNa, CHK2, MET, LCK, SRC, GSK3β, Erk2, PKACα, AKT2, INSR, p38α, AKT1, and MSK1. In this first embodiment, enzymes **352** are disposed within columns 1 through 24 in the exact order illustrated in **FIG. 3****.** In a second illustrative embodiment, kinases 1 through 24 comprise enzymes **452** illustrated in **FIG. 4****.** Enzymes **452** include kinases PKCβ2, ROCK2, CDK2, MST2, PKG1α, PAK2, IGF1R, FGFR1, MARK1, CAMK2δ, PIM2, BTK, c-TAK1, DYRK1a, CaMK4, AMPKα1, FLT3, HGK, KDR, Raf-1, P70S6K, IRAK4, SGK, and SYK. In this second embodiment, enzymes **452** are disposed within columns 1 through 24 in the exact order illustrated in **FIG. 4****.** While 24 columns of kinases are illustrated in **FIGS. 1-3****,** this is not intended to limit the invention. Other enzymes may be used, and the number of columns may be other than 24. The enzyme plate may also include a plurality of control wells as determined by the particular application.

Substrate plate **160** includes a plurality of enzyme substrates to be added to the enzyme plate. Each well of substrate plate **160** includes a solution containing a peptide, ATP (adenosine 5'-triphosphate), and a cofactor. Each enzyme acts on a specific peptide sequence. **FIGS. 3** and **4** illustrate the peptide sequences **354, 454** for each corresponding enzyme **352, 452,** respectively. **FIGS. 3** and **4** also illustrate the specific concentration of ATP **356, 456** and the specific cofactor and cofactor concentration **358, 458** required for each enzyme **352, 452.**

Kits in accordance with the present invention typically include all components necessary for screening a compound for enzyme inhibition activity, with the exception, of course, of the compound(s) of interest to be tested. For example, the kit may contain controls as indicated in **FIG. 2****,** a reconstitution buffer **152,** DTT **154** for addition to the reconstitution buffer prior to addition of the buffer to the enzyme plate, a protease inhibitor **156** also for addition to the reconstitution buffer prior to addition of the buffer to the enzyme plate, and a termination buffer **158** that is added to the enzyme plate to terminate the enzyme reaction prior to reading of the plate.

Essentially any kinase is suitable for use in the kits of the present invention, including those comprising EC (Enzyme Commission) numbers such as 2.1, 2.7, 2.8, 3.1, 3.4, 4.1, 6.2, or the like. These include, for example, a protein kinase, a protein kinase A, a protein kinase B, a protein kinase C, a hexokinase, a phosphofructokinase, a phosphoglycerate kinase, a pyruvate kinase, a cyclic AMP-dependent protein kinase, a cyclic GMP-dependent protein kinase, a calmodulin-dependent protein kinase II, a casein kinase I, a casein kinase II, a glycogen synthase kinase-3, a cyclin-dependent kinase (e.g., CDK2, CDK4, CDK6, or the like), a p34/cdc2 kinase, a nucleic acid kinase, or the like. **FIGS. 3** and **4** provide examples of two kinase panels particularly suitable for use in the present invention.

In addition, the present invention may be adapted to other enzymes such as, for example phosphatases, proteases, and histone deacetylases (HDACs). In those embodiments, the materials to be separated include, e.g., a phosphatase enzyme substrate and product, a dephosphorylated product, or the like. Essentially any phosphatase is suitable for use in the assays of the present invention, just one example being those comprising the EC number 3.1.3. To further illustrate, phosphatase enzymes that are optionally used in the assays described herein include, e.g., a protein phosphatase, an acid phosphatase, an alkaline phosphatase, a sugar phosphatase, a polynucleotide phosphatase, or the like.

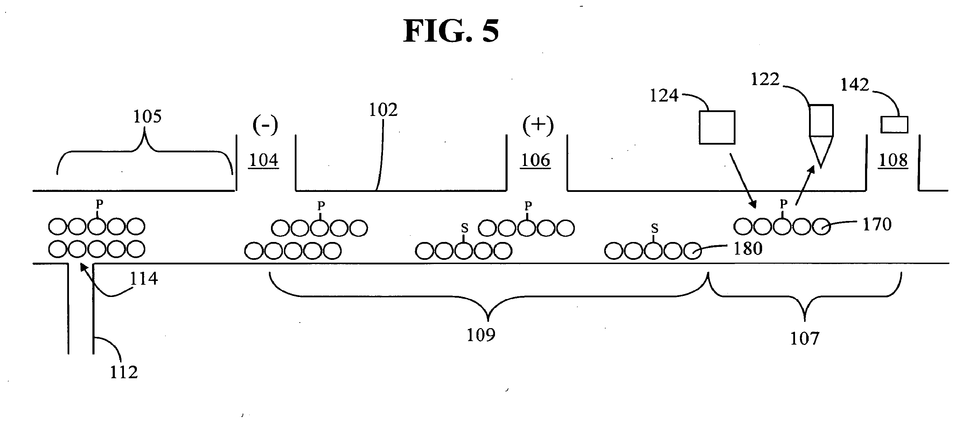

Another aspect of the invention is a system for screening a compound for enzyme inhibition activity. **FIGS. 1** and **5****,** which are not to scale, illustrate one embodiment of a system **100** for screening compounds for enzyme activity, e.g., protein kinase activity, in accordance with the present invention. Note that the system is also suitable for use with other enzymes such as, for example, phosphatases, proteases, and histone deacetylases (HDACs).

System **100** includes a microfluidic device **110,** a computer **120,** a detector **122,** a controller **130,** and a fluid direction system **140.** The system additionally includes a kit such as has been described above, including first and second multiwell plates **150** and **160,** reconstitution buffer **152,** DTT **154,** protease inhibitor **156,** and termination buffer **158.**

Microfluidic device **110** includes at least one microchannel **102** and a plurality of reservoirs or ports **104, 106, 108** in fluid communication with microchannel **102.** Aliquots of a sample containing a test compound (prepared in first and second multiwell plates **150** and **160**) are flowed into main microchannel **102** from capillary element **112** towards reservoir **108** by, for example, applying a vacuum at reservoir **108** (or another point in the system) and/or by applying appropriate voltage gradients. As used herein, a "capillary element" includes an elongated body structure having a channel (e.g., a microchannel) disposed therethrough. A capillary element is preferably an integral extension of the body structure of a single microfluidic device, as illustrated in **FIG. 1****,** but may also be a separate component that is temporarily coupled to one or more microfluidic device body structures. A microfluidic device in accordance with the present invention may include a single capillary element as illustrated in **FIG. 1** or may include a plurality of capillary elements (e.g., 4 or 12).

Additional materials, such as buffer solutions, substrate solutions, enzyme solutions, and the like, are optionally flowed from reservoirs **104** or **106** and into main microchannel **102.** Flow from these reservoirs is optionally performed by modulating fluid pressure, by electrokinetic approaches, or both (as illustrated in **FIG. 5**)**.** A number of possible arrangements of channels within microfluidic device **110** are possible, the arrangement depending on the specific application. Channels within the microfluidic device are covered, as detailed below under the heading "Microfluidic Devices."

Detector **122** is in sensory communication with main microchannel **102,** detecting signals resulting, for example, from labeled materials flowing through the detection region **107.** Detector **122** is optionally in sensory communication with any of the channels or regions of the device where detection is desired. As used herein, the phrase "in sensory communication" refers to positioning of a detector such that it is operably connected to the channel, i.e., capable of receiving a detectable signal from the contents of a channel. In the case of optical signals, this requires only that the detector be positioned to receive the optical signal. Detector **122** is also operably connected to computer **120,** which digitizes, stores, and manipulates signal information detected by detector **122,** for example, using any instruction set for determining enzyme activity, concentration, molecular weight or identity, or the like.

Fluid direction system **140** controls pressure, voltage, or both, e.g., at the reservoirs of the system or through the channels of the system, or at vacuum couplings fluidly coupled to main microchannel **102** or other channels described above. Optionally, as depicted, computer **120** controls fluid direction system **140.** In one set of embodiments, computer **120** uses signal information to select further parameters for the microfluidic system. In certain embodiments, controller **130** dispenses aliquots of buffers or other materials into, for example, main microchannel **102.** In these embodiments, controller **130** is also operably connected to computer **120,** which directs controller **130** function.

Although not shown, a microfluidic device handler is also typically included in the integrated systems of the present invention. Microfluidic device handlers generally control, e.g., the x-y-z translation of microfluidic device **110** relative to multiwell plate **150** or other system components, under the direction of computer **120,** to which the device handlers are operably connected. Microfluidic device handlers may also be involved in the transfer of materials from multiwell plate **160** to multiwell plate **150.** Microfluidic devices, computers, detectors, controllers, and fluid flow systems in accordance with the present invention are described in more detail below.

Yet another aspect of the present invention provides a method of screening a compound for enzyme inhibition activity. A sample mixture is obtained using a kit such as has been described above, the sample mixture comprising an enzyme, an enzyme substrate, a test compound and a product. The sample mixture is provided to a microfluidic device such as has been described above. Vacuum pressure is applied to the sample mixture, flowing the sample mixture along a microchannel of the microfluidic device, thereby separating the sample mixture to produce separated materials. The separated materials are detected, and enzyme inhibition activity is determined based on the detection of the separated materials.

The compounds to be screened are prepared using a multiwell compound plate in addition to enzyme and substrate plates such as those illustrated at **150** and **160,** respectively. The enzyme and substrate plates are stored frozen and are thawed prior to use.

Prepared compounds are added to enzyme plate **150** from the compound plate and incubated for a predetermined length of time as determined by the specific application. Reconstitution buffer **152,** combined with DTT **154** and a protease inhibitor **156,** is added to the enzyme plate either before adding the prepared compounds or in combination with the compounds. After expiration of the predetermined length of time, a portion of a substrate solution corresponding with a specific enzyme is removed from a well of substrate plate **160** and added to the corresponding well of enzyme plate **150.** This mixture is left to incubate for a predetermined length of time. After expiration of this time, termination buffer **158** is added to each well of enzyme plate **150.** See **Example 1,** below, for detailed steps. Aliquots of sample from each well are then provided to microfluidic device **110** for separation and analysis.

Each sample is flowed into region **105** of microchannel **102** via capillary element **112** by applying negative fluid pressure to the materials via a vacuum source applied to port **108.** The materials are flowed into a microchannel separation region **109.** Under an applied voltage, phosphorylated product is separated from unreacted substrate based upon a difference in mobilities of the phosphorylated product and the unreacted substrate. The separation region preferably contains free solution but may include an ion-exchange material (e.g., a polyarginine, a polylysine, a modified polyacrylamide, a modified dimethylacrylamide, or the like). Optionally, the ion-exchange material includes a polyacrylamide or a dimethylacrylamide modified (e.g., via covalent attachment, adsorption, or the like) by one or more anionic or cationic additives. In certain embodiments, the ion-exchange material is covalently or otherwise attached to a plurality of microbeads or a gel. In addition, the methods optionally include flowing the ion-exchange material into the separation region. The methods include detecting the resulting separated product and the unreacted substrate.

The sample is flowed through microchannel **102** and past detector **122.** As the sample nears detector **122,** a beam of light from LED (or laser or other light source) **124** is directed towards the sample. Fluorescence from the sample is then detected by detector **122.** Signals from detector **122** are transmitted to computer **120** for analysis and determination of the level of enzyme inhibition activity, if any, caused by the compound of interest.

In certain embodiments, more than one product is formed by contacting the first and second materials. These are also optionally separated according to the methods described herein. The methods optionally include flowing materials in the microfluidic device in the absence of an applied electric field, or flowing materials in the microfluidic device under a simultaneously applied electric field.

**FIG. 5** schematically illustrates one embodiment of an ion-exchange-induced mobility-shift-based separation method that is used for high-throughput compound screening in accordance with the present invention. As shown, microchannel configuration **100** includes microchannel **102** into which a sample including enzyme and substrate solutions is placed via capillary element **112.** Under negative pressure applied by a vacuum **142** at vacuum port **108,** the sample is continuously flowed from sample port **114** of main microchannel **102** towards port **108** and past detector **122** to detect a reaction product **170** and unreacted substrate **180.** Optionally, a modulator, an inhibitor, an antagonist, an agonist, or other material may be flowed from a well on a multiwell plate or another external source through capillary element **112** into region **105** of main microchannel **102,** e.g., to assess its impact on the assay.

A free solution or a solution of a suitable ion exchanger or other chromatographic material, as appropriate, is optionally placed into microchannel **102** and flowed continuously into separation region **109** of main microchannel **102** during the course of the assay to effect separation of the reaction product from unreacted substrate based upon their, e.g., respective mobilities or other distinguishing properties. Alternatively, separation region **109** of main microchannel **102** is selectively modified by, for example, coating it with the ion exchanger prior to commencing the assay. An additional option includes manufacturing at least separation region **109** of main microchannel **102** to possess the desired ion-exchange characteristics (e.g., by selecting appropriate microfluidic device substrate materials).

The method of screening a compound for enzyme inhibition activity includes mixing an enzyme solution (e.g., a protein kinase, a protein kinase A, a nucleic acid kinase, or the like) and a substrate solution to produce a phosphorylated product such as product **170** illustrated in **FIG. 5****.**

### EXAMPLE 1

The following example serves to illustrate, but not to limit, the present invention.

### Materials

A DeskTop Profiler™ instrument (available from Caliper Life Sciences, Inc.); ProfilerPro™ enzyme and substrate plates (available from Caliper Life Sciences, Inc.); a 384-well plate for control/compound preparation; reconstitution buffer, ATP/peptides, termination buffer, DTT, protease inhibitor, and DMSO; and microfluidic devices for performing mobility shift assays were used. The 384-well ProfilerPro™ enzyme plates include 24 enzymes per plate, pre-dispensed in columns of 16. The 384-well ProfilerPro™ substrate plates include 24 peptide/ATP pairs per plate, pre-dispensed in columns of 16. Prior to use, the plates are stored below -70 °C. Buffers are stored below 4 °C. DTT and protease inhibitor are stored below -70 °C.

### Procedure

1. Prepare components.
   a. Remove reconstitution buffer and termination buffer from freezer and thaw. If stored for extended periods prior to use, maintain at 4 °C.
   b. To 50 mL reconstitution buffer, add 50 µL of 1M DTT and 500 µL of 100x protease inhibitor.
   c. Prepare compounds.
      i. For 1 µL compound transfer, dilute compounds to 26x assay concentration in 100% DMSO; place in compound plate in rows C through N (see FIG. 2).
         **OR**
      ii. For 16 µL compound transfer, dilute compounds to 1.625x assay concentration in previously prepared reconstitution buffer with DTT and protease inhibitor; place in compound plate in rows C through N (see FIG. 2).
   d. Prepare controls.
      i. For 1 µL control transfer, add 100% DMSO to compound plate in rows A, B, O, and P (see FIG. 2).
         **OR**
      ii. For 16 µL control transfer, make 6.25% DMSO solution in previously prepared reconstitution buffer with DTT and protease inhibitor; place in compound plate in rows A, B, O, and P (see FIG. 2).
   e. Place reconstitution buffer, compound plate, and termination buffer in incubator at 28 °C.
2. Prepare ProfilerPro™ substrate and enzyme plates.
   a. Remove substrate plate from freezer and incubate at 28 °C.
   b. Wait 30 minutes
   c. Remove enzyme plate from freezer and incubate at 28 °C; after 15 minutes, spin enzyme plate at 1000 rpm for 1 minute if bubbles are observed; remove seal.
   d. Transfer compound/control from the compound plate to the enzyme plate.
      i. For 1 µL compound/control transfer, first add 15 µL of previously prepared reconstitution buffer with DTT and protease inhibitor to each well of the enzyme plate and mix; then add 1 µL compound/control from each well of the compound plate to a separate well of the enzyme plate; mix.
         **OR**
      ii. For 16 µL compound/control transfer, add 16 µL of the previously prepared mixture of compound/control and reconstitution buffer (with DTT and protease inhibitor) to each well of the enzyme plate and mix.
   e. Pre-incubate the enzyme plate for up to 15 minutes at 28 °C.
   f. At least 60 minutes after placing the substrate plate in the incubator in Step a, remove the substrate plate from the incubator and spin at 500 rpm for 1 minute if bubbles are observed; remove seal.
   g. Transfer 10 µL of substrate from each well of the substrate plate to each well of the enzyme plate; spin at 1000 rpm for 1 minute if bubbles are observed.
   h. Cover enzyme plate and incubate for 90 minutes at 28 °C.
   i. Add 45 µL of termination buffer to each well of the enzyme plate in the same order used when adding substrate (to avoid variable incubation time); mix if necessary; spin at 1000 rpm for 1 minute.
3. Read the enzyme plate using the DeskTop Profiler™ instrument.
   All assays are run at ATP/Km =1 for each enzyme. Common buffers are used:
   *Reaction Buffer*
      100 mM HEPES, pH = 7.5
      10 mM MgCl₂ or MnCl₂
      0.003% Brij-35
      1 mM DTT
      4% DMSO
      [E] is predetermined to yield the appropriate conversion upon incubation and termination.
   *Separation Buffer*
      100 mM HEPES, pH = 7.5
      0.015% Brij-35
      1 mM EDTA
      0.1 % Coating Reagent 3
      5% DMSO

### Results

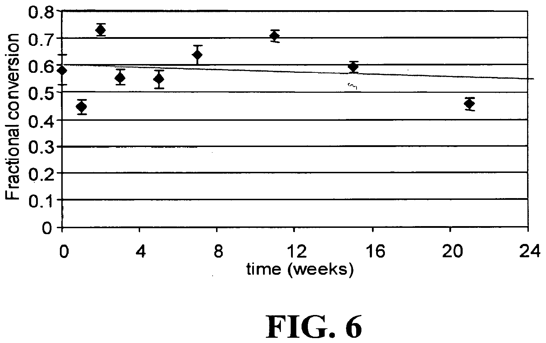

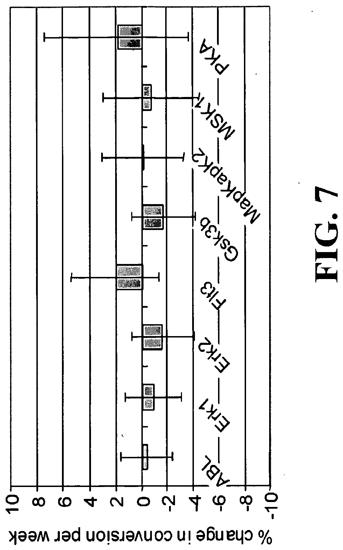

*Enzyme degradation.* Enzymes were diluted to 260x the assay concentration in a storage buffer and 100 nL dispensed into the wells of a 384-well microtiter plate. Plates were frozen and stored at -80 °C. Enzymes were assayed over 22 weeks by thawing and adding 16 µL of reconstitution buffer and 10 µL of substrate. After a 1-hour incubation, the reactions were quenched and run on a Caliper Life Sciences, Inc., LabChip® 3000 Drug Discovery System. Sample data are shown for ABL in **FIG. 6****.** **FIG. 7** shows the change in conversion per week for a subset of the enzymes that was tested for 22 weeks (error bars = 95% confidence intervals). No enzyme showed a statistically significant difference from no change in activity.

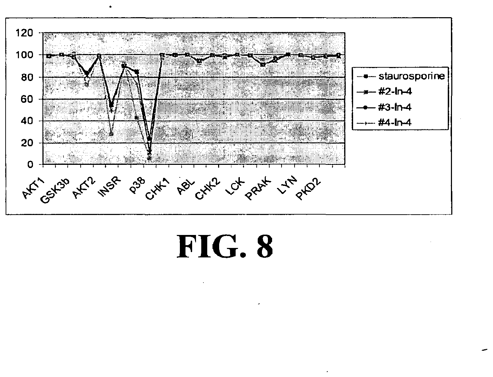

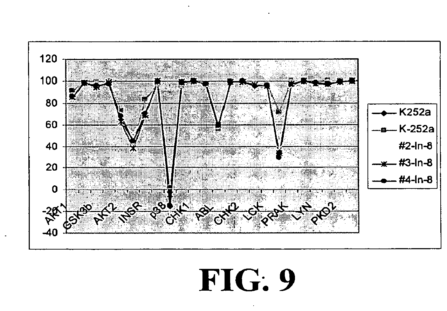

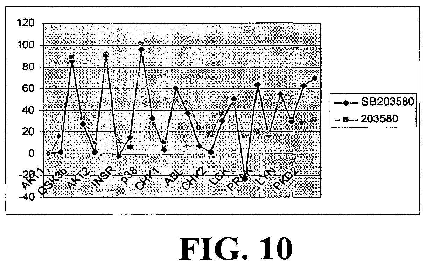

*Kinase Selectivity Screening.* A set of 13 commercially available kinase inhibitors was run at a concentration of 10 µM against a ProfilerPro™ enzyme plate. In this example, the enzymes were disposed within columns 1 through 24 in the order of AKT1, MSK1, GSK3β, Erk1, AKT2, CK1δ, INSR, Erk2, p38, MET, CHK1, SRC, ABL, RSK1, CHK2, FYN, LCK, PKCζ, PRAK, AurA, LYN, MAPKAPK2, PKD2, PKA. Selectivity data for staurosporine **(****FIG. 8****),** K252A **(****FIG. 9****),** and SB203580 **(****FIG. 10****)** were run at Caliper Life Sciences, Inc. and at an external site, showing good agreement.

*Reproducibility.* Two ProfilerPro™ plates from two batches were run each of three days with DMSO controls in 5 wells. An ANOVA revealed an overall CV of 18.8% with the following sources of variability (% of total):

| | |
|---|---|
| day-to-day | 13.9% |
| batch-to-batch | 0% |
| plate-to plate | 26.5% |
| assay | 59.6% |

### MICROFLUIDIC DEVICES

Many different microscale systems are optionally adapted for use in the methods of the present invention. These systems are described in numerous publications by the inventors and their coworkers, including certain issued U.S. Patents, such as U.S. Patent Nos. 5,699,157 (J. Wallace Parce) issued 12/16/97, 5,779,868 (J. Wallace Parce et al.) issued 07/14/98, 5,800,690 (Calvin Y.H. Chow et al.) issued 09/01/98, 5,842,787 (Anne R. Kopf-Sill et al.) issued 12/01/98, 5,852,495 (J. Wallace Parce) issued 12/22/98, 5,869,004 (J. Wallace Parce et al.) issued 02/09/99, 5,876,675 (Colin B. Kennedy) issued 03/02/99, 5,880,071 (J. Wallace Parce et al.) issued 03/09/99, 5,882,465 (Richard J. McReynolds) issued 03/16/99, 5,885,470 (J. Wallace Parce et al.) issued 03/23/99, 5,942,443 (J. Wallace Parce et al.) issued 08/24/99, 5,948,227 (Robert S. Dubrow) issued 09107/99, 5,955,028 (Calvin Y.H. Chow) issued 09/21/99, 5,957,579 (Anne R. Kopf-Sill et al.) issued 09/28/99, 5,958,203 (J. Wallace Parce et al.) issued 09/28/99, 5,958,694 (Theo T. Nikiforov) issued 09/28/99, 5,959,291 (Morten J. Jensen) issued 09/28/99, 5,964,995 (Theo T. Nikiforov et al.) issued 10/12/99, 5,965,001 (Calvin Y. H. Chow et al.) issued 10/12/99, 5,965,410 (Calvin Y. H. Chow et al.) issued 10/12/99, 5,972,187 (J. Wallace Parce et al.) issued 10/26/99, 5,976,336 (Robert S. Dubrow et al.) issued 11/2/99, 5,989,402 (Calvin Y. H. Chow et al.) issued 11/23/99, 6,001,231 (Anne R. Kopf-Sill) issued 12/14/99, 6,011,252 (Morten J. Jensen) issued 1/4/00, 6,012,902 (J. Wallace Parce) issued 1/11/00, 6,042,709 (J. Wallace Parce et al.) issued 3/28/00, 6,042,710 (Robert S. Dubrow) issued 3/28/00, 6,046,056 (J. Wallace Parce et al.) issued 4/4/00, 6,048,498 (Colin B. Kennedy) issued 4/11/00, 6,068,752 (Robert S. Dubrow et al.) issued 5/30/00, 6,071,478 (Calvin Y. H. Chow) issued 6/6/00, 6,074,725 (Colin B. Kennedy) issued 6/13/00, 6,080,295 (J. Wallace Parce et al.) issued 6/27/00, 6,086,740 (Colin B. Kennedy) issued 7/11/00, 6,086,825 (Steven A. Sundberg et al.) issued 7/11/00, 6,090,251 (Steven A. Sundberg et al.) issued 7/18/00, 6,100,541 (Robert Nagle et al.) issued 8/8/00, 6,107,044 (Theo T. Nikiforov) issued 8/22/00, 6,123,798 (Khushroo Gandhi et al.) issued 9/26/00, 6,129,826 (Theo T. Nikiforov et al.) issued 10/10/00, 6,132,685 (Joseph E. Kersco et al.) issued 10/17/00, 6,148,508 (Jeffrey A. Wolk) issued 11/21/00, 6,149,787 (Andrea W. Chow et al.) issued 11/21/00, 6,149,870 (J. Wallace Parce et al.) issued 11/21/00, 6,150,119 (Anne R. Kopf-Sill et al.) issued 11/21/00, 6,150,180 (J. Wallace Parce et al.) issued 11/21/00, 6,153,073 (Robert S. Dubrow et al.) issued 11/28/00, 6,156,181 (J. Wallace Parce et al.) issued 12/5/00, 6,167,910 (Calvin Y. H. Chow) issued 1/2/01, 6,171,067 (J. Wallace Parce) issued 1/9/01, 6,171,850 (Robert Nagle et al.) issued 1/9/01, 6,172,353 (Morten J. Jensen) issued 1/9/01, 6,174,675 (Calvin Y. H. Chow et al.) issued 1/16/01, 6,182,733 (Richard J. McReynolds) issued 2/6/01, 6,186,660 (Anne R. Kopf-Sill et al.) issued 2/13/01, 6,221,226 (Anne R. Kopf-Sill) issued April 24, 2001, 6,233,048 (J. Wallace Parce) issued May 15, 2001, 6,235,175 (Robert S. Dubrow et al.) issued 5/22/01, 6,235,471 (Michael Knapp et al.) issued 5/22/01, 6,238,538 (J. Wallace Parce et al.) issued 5/29/01, 6,251,343 (Robert S. Dubrow et al.) issued 6/26/01, 6,267,858 (J. Wallace Parce et al.) issued 7/31/01, 6,274,089 (Andrea W. Chow et al.) issued 8/14/01, 6,274,337 (J. Wallace Parce et al.) issued 8/14/01, 6,287,520 (J. Wallace Parce et al.) issued 9/11/01, 6,287,774 (Theo T. Nikiforov) issued 9/11/01, 6,303,343 (Anne R. Kopf-Sill) issued 10/16/01, 6,306,590 (Tammy Burd Mehta et al.) issued 10/23/01, and 6,306,659 (J. Wallace Parce et al.) issued 10/23/01.

Systems that are optionally adapted for use in the methods of the present invention are also described in, e.g., various published PCT applications, including WO 98/00231, WO 98/00705, WO 98/00707, WO 98/02728, WO 98/05424, WO 98/22811, WO 98/45481, WO 98/45929, WO 98/46438, and WO 98/49548, WO 98/55852, WO 98/56505, WO 98/56956, WO 99/00649, WO 99/10735, WO 99/12016, WO 99/16162, WO 99/19056, WO 99/19516, WO 99/29497, WO 99/31495, WO 99/34205, WO 99/43432, WO 99/44217, WO 99/56954, WO 99/64836, WO 99/64840, WO 99/64848, WO 99/67639, WO 00/07026, WO 00/09753, WO 00/10015, WO 00/21666, WO 00/22424, WO 00/26657, WO 00/42212, WO 00/43766, WO 00/45172, WO 00/46594, WO 00/50172, WO 00/50642, WO 00/58719, WO 00/60108, WO 00/70080, WO 00/70353, WO 00/72016, WO 00/73799, WO 00/78454, WO 01/02850, WO 01/14865, WO 01/17797, and WO 01/27253.

The methods of the invention are generally performed within fluidic channels. In some cases, as mentioned above, the channels are simply present in a capillary or pipettor element, e.g., a glass, fused silica, quartz, or plastic capillary. The capillary element is fluidly coupled to a source of, e.g., the reagent, sample, modulator, or other solution (e.g., by dipping the capillary element into a well on a microtiter plate), which is then flowed along the channel (e.g., a microchannel) of the element. The term "microfluidic," as used herein, generally refers to one or more fluid passages, chambers or conduits which have at least one internal cross-sectional dimension, e.g., depth, width, length, diameter, etc., that is less than 1 mm, and typically between about 0.1 µm and about 500 µm.

In the devices of the present invention, the microscale channels or cavities typically have at least one cross-sectional dimension between about 0.1 µm and 200 µm, preferably between about 0.1 µm and 100 µm, and often between about 0.1 µm and 50 µm. Accordingly, the microfluidic devices or systems prepared in accordance with the present invention typically include at least one microscale channel, usually at least two intersecting microscale channels, and often, three or more intersecting channels disposed within a single body structure that make up a channel network. Channel intersections may exist in a number of formats, including cross intersections, "Y" and/or "T" intersections, or any number of other structures whereby two channels are in fluid communication.

The body structures of the microfluidic devices described herein arc typically manufactured from two or more separate portions or substrates which when appropriately mated or joined together, form the microfluidic device of the invention, e.g., containing the channels and/or chambers described herein. During body structure fabrication, the microfluidic devices described herein will typically include a top portion, a bottom portion, and an interior portion, wherein the interior portion substantially defines the covered channels and chambers of the device.

In one aspect, a bottom portion of the unfinished device includes a solid substrate that is substantially planar in structure, and which has at least one substantially flat upper surface. Channels are typically fabricated on one surface of the device and sealed (i.e., covered) by overlaying the channels with an upper substrate layer. A variety of substrate materials are optionally employed as the upper or bottom portion of the device. Typically, because the devices are microfabricated, substrate materials will be selected based upon their compatibility with known microfabrication techniques, e.g., photolithography, wet chemical etching, laser ablation, air abrasion techniques, LIGA, reactive ion etching, injection molding, embossing, and other techniques. The substrate materials are also generally selected for their compatibility with the full range of conditions to which the microfluidic devices may be exposed, including extremes of pH, temperature, electrolyte concentration, and/or for their chromatographic properties. Accordingly, in some preferred aspects, the substrate material may include materials normally associated with the semiconductor industry in which such microfabrication techniques are regularly employed, including, e.g., silica-based substrates, such as glass, quartz, silicon or polysilicon, as well as other substrate materials, such as gallium arsenide and the like. In the case of semiconductive materials, it will often be desirable to provide an insulating coating or layer, e.g., silicon oxide, over the substrate material, and particularly in those applications where electric fields are to be applied to the device or its contents.

In additional preferred aspects, the substrate materials will comprise polymeric materials, e.g., plastics, such as polymethylmethacrylate (PMMA), polycarbonate, polytetrafluoroethylene (TEFLON™), polyvinylchloride (PVC), polydimethylsiloxane (PDMS), polysulfone, polystyrene, polymethylpentene, polypropylene, polyethylene, polyvinylidine fluoride, ABS (acrylonitrile-butadiene-styrene copolymer), and the like. In preferred embodiments, at least the separation region(s) is/are fabricated from polyacrylamide, dimethylacrylamide, modified versions thereof, nonionic detergents, ionic detergents, or the like. Such polymeric substrates are readily manufactured using available microfabrication techniques, as described above, or from microfabricated masters, using known molding techniques, such as injection molding, embossing or stamping, or by polymerizing the polymeric precursor material within the mold (*see,* e.g., U.S. Patent No. 5,512,131). Such polymeric substrate materials are preferred for their ease of manufacture, low cost and disposability, as well as their general inertness to most extreme reaction conditions. Again, these polymeric materials optionally include treated surfaces, e.g., derivatized or coated surfaces, to enhance their utility in the microfluidic system, e.g., to provide enhanced fluid direction, e.g., as described in U.S. Pat. No. 5,885,470 (J. Wallace Parce et al.) issued 3/23/99.

The channels and/or cavities of the microfluidic devices are typically fabricated into the upper surface of the bottom substrate or portion of the device, as microscale grooves or indentations, using the above described microfabrication techniques. The top portion or substrate also comprises a first planar surface, and a second surface opposite the first planar surface. In the microfluidic devices prepared in accordance with certain aspects of the methods described herein, the top portion can include at least one aperture, hole, or port disposed therethrough, e.g., from the first planar surface to the second surface opposite the first planar surface. In other embodiments, the port(s) is/are optionally omitted, e.g., where fluids are introduced solely through external capillary elements.

The first planar surface of the top portion or substrate is then mated, e.g., placed into contact with, and bonded to the planar surface of the bottom substrate, covering and sealing the grooves and/or indentations in the surface of the bottom substrate, to form the channels and/or chambers (i.e., the interior portion) of the device at the interface of these two components. Bonding of substrates is typically carried out by any of a number of different methods, e.g., thermal bonding, solvent bonding, ultrasonic welding, and the like. The finished body structure of a device is a unitary structure that houses, e.g., the channels and/or chambers of the device.

The hole(s) in the top of the finished device is/are oriented to fluidly communicate with at least one of the channels and/or cavities. In the completed device, the hole(s) optionally function as reservoirs for facilitating fluid or material introduction into the channels or chambers of the device, as well as providing ports at which, e.g., pressure elements (e.g., vacuum sources, etc.) are optionally placed into contact with fluids within the device, allowing application of pressure gradients along the channels of the device to control and direct fluid transport within the device. In optional embodiments, extensions are provided over these reservoirs to allow for increased fluid volumes, permitting longer running assays, and better controlling fluid flow parameters, e.g., hydrostatic pressures. Examples of methods and apparatus for providing such extensions are described in U.S. Patent No. 6,251,343, filed February 24, 1998. These devices are optionally coupled to a sample introduction port, e.g., a pipettor or capillary element, which serially introduces multiple samples, e.g., from the wells of a microtiter plate. Thus, in some embodiments, both reservoirs in the upper surface and external capillary elements are present in a single device.

The sources of reagents, enzymes, substrates, samples, eluents, separation buffers, and other materials are optionally fluidly coupled to the microchannels in any of a variety of ways. In particular, those systems comprising sources of materials set forth in Knapp et al. "Closed Loop Biochemical Analyzers" (WO 98/45481; PCT/US98/06723) and U.S. Pat. No. 5,942,443 issued August 24, 1999, entitled "High Throughput Screening Assay Systems in Microscale Fluidic Devices" to J. Wallace Parce et al. and, e.g., in 60/128,643 filed April 4, 1999, entitled "Manipulation of Microparticles In Microfluidic Systems," by Mehta et al. are applicable.

In these systems and as noted above, a capillary or pipettor element (i.e., an element in which components are optionally moved from a source to a microscale element such as a second channel or reservoir) is temporarily or permanently coupled to a source of material. The source is optionally internal or external to a microfluidic device that includes the pipettor or capillary element. Example sources include microwell plates, membranes or other solid substrates comprising lyophilized components, wells or reservoirs in the body of the microscale device itself and others.

### FLOW OF MATERIALS IN MICROFLUIDIC SYSTEMS

A preferred method of flowing materials along the microchannels or other cavities of the devices described herein is by pressure-based flow. Pressure is applied with or without a simultaneously applied electric field. Application of a pressure differential along a channel is carried out by any of a number of approaches. For example, it may be desirable to provide relatively precise control of the flow rate of materials, e.g., to precisely control incubation or separation times, etc. As such, in many preferred aspects, flow systems that are more active than hydrostatic pressure driven systems are employed. In certain cases, reagents may be flowed by applying a pressure differential across the length of the analysis channel. For example, a pressure source (positive or negative) is applied at the reagent reservoir at one end of the analysis channel, and the applied pressure forces the reagents/samples through the channel. The pressure source is optionally pneumatic, e.g., a pressurized gas, or a positive displacement mechanism, i.e., a plunger fitted into a reagent reservoir, for forcing the reagents through the analysis channel. Alternatively, a vacuum source is applied to a reservoir at the opposite end of the channel to draw the reagents through the channel. *See,* FIGS. 1 and 5. Pressure or vacuum sources may be supplied external to the device or system, e.g., external vacuum or pressure pumps sealably fitted to the inlet or outlet of the analysis channel, or they may be internal to the device, e.g., microfabricated pumps integrated into the device and operably linked to the analysis channel. Examples of microfabricated pumps have been widely described in the art. *See,* e.g., published International Application No. WO 97/02357.

The reagents are deposited in a reservoir or well at one end of an analysis channel and at a sufficient volume or depth, that the reagent sample creates a hydrostatic pressure differential along the length of the analysis channel, by virtue of it having greater depth than a reservoir at an opposite terminus of the channel. The hydrostatic pressure then causes the reagents to flow along the length of the channel. The reservoir volume is quite large in comparison to the volume or flow through rate of the channel, e.g., 10 µl reservoirs, vs. 1000 µm² channel cross-section. As such, over the time course of the assay/separation, the flow rate of the reagents will remain substantially constant, as the volume of the reservoir, and thus, the hydrostatic pressure changes very slowly. Applied pressure is then readily varied to yield different reagent flow rates through the channel. In screening applications, varying the flow rate of the reagents is optionally used to vary the incubation time of the reagents. In particular, by slowing the flow rate along the channel, one can effectively lengthen the amount of time between introduction of reagents and detection of a particular effect. Alternatively, analysis channel lengths, detection points, or reagent introduction points are varied in fabrication of the devices, to vary incubation times. *See also,* "Multiport Pressure Control System," by Chien and Parce, USSN 60/184,390, filed February 23, 2000, which describes multiport pressure controllers that couple pumps to multiple device reservoirs.

In further alternate aspects, hydrostatic, wicking and capillary forces are additionally, or alternately, used to provide for fluid flow. *See,* e.g., "Method and Apparatus for Continuous Liquid Flow in Microscale Channels Using Pressure Injection, Wicking and Electrokinetic Injection," by Alajoki et al., USSN 09/245,627, filed February 5, 1999. In these methods, an adsorbent material or branched capillary structure is placed in fluidic contact with a region where pressure is applied, thereby causing fluid to move towards the adsorbent material or branched capillary structure.

In alternative aspects, flow of reagents is driven by inertial forces. In particular, the analysis channel is optionally disposed in a substrate that has the conformation of a rotor, with the analysis channel extending radially outward from the center of the rotor. The reagents are deposited in a reservoir that is located at the interior portion of the rotor and is fluidly connected to the channel. During rotation of the rotor, the centripetal force on the reagents forces the reagents through the analysis channel, outward toward the edge of the rotor. Multiple analysis channels are optionally provided in the rotor to perform multiple different analyses. Detection of a detectable signal produced by the reagents is then carried out by placing a detector under the spinning rotor and detecting the signal as the analysis channel passes over the detector. Examples of rotor systems have been previously described for performing a number of different assay types. *See,* e.g., Published International Application No. WO 95/02189. Test compound reservoirs are optionally provided in the rotor, in fluid communication with the analysis channel, such that the rotation of the rotor also forces the test compounds into the analysis channel.

For purposes of illustration, the discussion has focused on a single channel and accessing capillary; however, it will be readily appreciated that these aspects may be provided as multiple parallel analysis channels (e.g., each including mixing and separation regions) and accessing capillaries, in order to substantially increase the throughput of the system. Specifically, single body structures may be provided with multiple parallel analysis channels coupled to multiple sample accessing capillaries that are positioned to sample multiple samples at a time from sample libraries, e.g., multiwell plates. As such, these capillaries are generally spaced at regular distances that correspond with the spacing of wells in multiwell plates, e.g., 9 mm centers for 96 well plates, 4.5 mm for 3 84 well plates, and 2.25 mm for 1536 well plates.

In alternate aspects, an applied pressure is accompanied by the simultaneous application of an electric field to further effect fluid transport, e.g., through the mixing and/or separation regions of the microchannel. The electrokinetic transport systems of the invention typically utilize electric fields applied along the length of microchannels that have a surface potential or charge associated therewith. When fluid is introduced into the microchannel, the charged groups on the inner surface of the microchannel ionize, creating locally concentrated levels of ions near the fluid surface interface. Under an electric field, this charged sheath migrates toward the cathode or anode (depending upon whether the sheath comprises positive or negative ions) and pulls the encompassed fluid along with it, resulting in bulk fluid flow. This flow of fluid is generally termed electroosmotic flow. Where the fluid includes reagents (e.g., materials to be separated), the reagents are also pulled along. A more detailed description of controlled electrokinetic material transport systems in microfluidic systems is described in published International Patent Application No. WO 96/04547.

### INTEGRATED SYSTEMS

The present invention, in addition to other integrated system components, also optionally includes a microfluidic device handler for performing the methods disclosed herein. Specifically, the microfluidic device handler includes a holder configured to receive the microfluidic device, a container sampling region proximal to the holder, and the controller. During operation of the handler, the controller directs, e.g., dipping of microfluidic device capillary or pipettor element(s) into a portion of, e.g., a microwell plate in the container sampling region. The microfluidic device handler also includes a computer or a computer readable medium operably connected to the controller. The computer or the computer readable medium includes an instruction set for varying or selecting a rate or a mode of dipping capillary element(s) into fluid materials.

Although the devices and systems specifically illustrated herein are generally described in terms of the performance of a few or one particular operation, it will be readily appreciated from this disclosure that the flexibility of these systems permits easy integration of additional operations into these devices. For example, the devices and systems described will optionally include structures, reagents and systems for performing virtually any number of operations in addition to the operations specifically described herein. Aside from fluid handling, assays, and separation of sample and/or reaction components, other upstream or downstream operations include, e.g., extraction, purification, amplification, cellular activation, labeling reactions, dilution, aliquotting, labeling of components, assays and detection operations, electrokinetic or pressure-based injection of components or materials into contact with one another, or the like. Assay and detection operations include, without limitation, cell fluorescence assays, cell activity assays, receptor/ligand assays, immunoassays, or the like.

In the present invention, the separated materials are optionally monitored and/or detected so that, e.g., an activity can be determined. The systems described herein generally include microfluidic device handlers, as described above, in conjunction with additional instrumentation for controlling fluid transport, flow rate and direction within the devices, detection instrumentation for detecting or sensing results of the operations performed by the system, processors, e.g., computers, for instructing the controlling instrumentation in accordance with preprogrammed instructions, receiving data from the detection instrumentation, and for analyzing, storing and interpreting the data, and providing the data and interpretations in a readily accessible reporting format.

### Controllers

A controller **130** of system **100** of the present invention directs dipping of capillary elements into, for example, multiwell plate **150** to sample reagents such as enzymes and substrates, fluid recirculation baths or troughs, or the like. A variety of controlling instrumentation is also optionally utilized in conjunction with the microfluidic devices and handling systems described herein, for controlling the transport, concentration, direction, and motion of fluids and/or separation of materials within the devices of the present invention, e.g., by pressure-based control.

As described above, in many cases, fluid transport, concentration, and direction are controlled in whole or in part, using pressure based flow systems that incorporate external or internal pressure sources to drive fluid flow. Internal sources include microfabricated pumps, e.g., diaphragm pumps, thermal pumps, and the like that have been described in the art. *See,* e.g., U.S. Patent Nos. 5,271,724, 5,277,556, and 5,375,979 and Published PCT Application Nos. WO 94/05414 and WO 97/02357. Preferably, external pressure sources are used, and applied to ports at channel termini. These applied pressures, or vacuums, generate pressure differentials across the lengths of channels to drive fluid flow through them. In the interconnected channel networks described herein, differential flow rates on volumes are optionally accomplished by applying different pressures or vacuums at multiple ports, or preferably, by applying a single vacuum at a common waste port (*see,* Figure 1) and configuring the various channels with appropriate resistance to yield desired flow rates. Example systems are also described in USSN 09/238,467 filed 1/28/99.

Typically, the controller systems are appropriately configured to receive or interface with a microfluidic device or system element as described herein. For example, the controller and/or detector, optionally includes a stage upon which the device of the invention is mounted to facilitate appropriate interfacing between the controller and/or detector and the device. Typically, the stage includes an appropriate mounting/alignment structural element, such as a nesting well, alignment pins and/or holes, asymmetric edge structures (to facilitate proper device alignment), and the like. Many such configurations are described in the references cited herein.

The controlling instrumentation discussed above is also used to provide for electrokinetic injection or withdrawal of material downstream of the region of interest to control an upstream flow rate. The same instrumentation and techniques described above are also utilized to inject a fluid into a downstream port to function as a flow control element.

### Detector

The devices described herein include signal detectors **122** which detect fluorescence. In other devices, signal detectors may detect concentration, phosphorescence, radioactivity, pH, charge, absorbance, refractive index, luminescence, temperature, magnetism, mass (e.g., mass spectrometry), or the like. The detector(s) optionally monitors one or a plurality of signals from upstream and/or downstream (e.g., in or proximal to the separation region) of an assay mixing point in which, e.g., a ligand and an enzyme are mixed. For example, the detector optionally monitors a plurality of optical signals which correspond in position to "real time" assay/separation results.

Example detectors or sensors include photomultiplier tubes, CCD arrays, optical sensors, temperature sensors, pressure sensors, pH sensors, conductivity sensors, mass sensors, scanning detectors, or the like. Materials which emit a detectable signal are optionally flowed past the detector, or, alternatively, the detector can move relative to the array to determine the position of an assay component (or, the detector can simultaneously monitor a number of spatial positions corresponding to channel regions, e.g., as in a CCD array). Each of these types of sensors is optionally readily incorporated into the microfluidic systems described herein. In these systems, such detectors are placed either within or adjacent to the microfluidic device or one or more channels, chambers or conduits of the device, such that the detector is within sensory communication with the device, channel, or chamber. The phrase "within sensory communication" of a particular region or element, as used herein, generally refers to the placement of the detector in a position such that the detector is capable of detecting the property of the microfluidic device, a portion of the microfluidic device, or the contents of a portion of the microfluidic device, for which that detector was intended. The detector optionally includes or is operably linked to a computer, e.g., which has software for converting detector signal information into assay result information (e.g., kinetic data of modulator activity), or the like. A microfluidic system optionally employs multiple different detection systems for monitoring the output of the system. Detection systems of the present invention are used to detect and monitor the materials in a particular channel region (or other reaction detection region).

The detector optionally exists as a separate unit, but is preferably integrated with the controller system, into a single instrument. Integration of these functions into a single unit facilitates connection of these instruments with the computer (described below), by permitting the use of few or a single communication port(s) for transmitting information between the controller, the detector, and the computer.

### Computer

As noted above, the microfluidic devices and integrated systems of the present invention include a computer **120** operably connected to the controller. The computer typically includes an instruction set, e.g., for varying or selecting a rate or a mode of dipping capillary or pipettor elements into fluid materials, for sampling fluidic materials (e.g., enzymes, substrates, reactants, chromatographic materials, eluents, separation buffers, etc.), or the like. Additionally, either or both of the controller system and/or the detection system is/are optionally coupled to an appropriately programmed processor or computer which functions to instruct the operation of these instruments in accordance with preprogrammed or user input instructions, receive data and information from these instruments, and interpret, manipulate and report this information to the user. As such, the computer is typically appropriately coupled to one or both of these instruments (e.g., including an analog to digital or digital to analog converter as needed).

The computer typically includes appropriate software for receiving user instructions, either in the form of user input into a set parameter fields, e.g., in a GUI, or in the form of preprogrammed instructions, e.g., preprogrammed for a variety of different specific operations. The software then converts these instructions to appropriate language for instructing the operation of the fluid direction and transport controller to carry out the desired operation, e.g., varying or selecting the rate or mode of fluid and/or microfluidic device movement, controlling flow rates within microscale channels, directing xyz translation of the microfluidic device or of one or more microwell plates, or the like. The computer then receives the data from the one or more sensors/detectors included within the system, and interprets the data, either provides it in a user understood format, or uses that data to initiate further controller instructions, in accordance with the programming, e.g., such as in monitoring and control of flow rates, temperatures, applied voltages, and the like. Additionally, the software is optionally used to control, e.g., pressure or electrokinetic modulated injection or withdrawal of material.

### EXAMPLE 2:

In vitro screening of kinase activity typically involves the use of a short peptide sequence used as an in vitro surrogate for the physiologically relevant protein substrate. An alternative embodiment of the invention may be employed to identify such substrate surrogates. Substrate plates for this purpose are arranged based on considerations similar to those taken into account for substrate plates that are matched with an enzyme plate as described above. I.e., the substrates are arranged based on factors such as, for example, electrophoretic mobility.

In an example assay, a kinase target of interest is incubated in a micro titer plate with a library of fluorescently labeled peptide substrate-candidates. The reaction is terminated and the mixture sipped onto a microfluidic chip. The extent of phosphorylation of each fluorescently labeled peptide is then measured by a mobility shift assay in accordance with the invention.

A control solution that contains only the peptide without any enzyme is provided for each peptide substrate-candidate. Thus, each multiwell plate includes two solutions containing each peptide, one solution without enzyme and one solution with enzyme. The control solution contains peptide, ATP, and reaction buffer. The enzyme solution contains the same components plus the enzyme. Both the control and enzyme-containing solutions are incubated for a predetermined period of time. At the end of that time, the reaction in the well with the enzyme-containing solution is terminated by the addition of an amount of termination buffer, while the same amount of separation buffer is added to the well containing the control solution. Addition of the same amount of material to both wells ensures that the final concentration of the labeled peptide in both wells is identical.

This example assay is carried out in a microfluidic device containing multiple external capillaries or "sippers" that allow samples from multiple sources to be simultaneously introduced to be processed in parallel within the microfluidic device. Examples of such microfluidic devices are provided in U.S. Patent No. 6,358,387, which is assigned to the assignee of this invention. The location of the control well on the multiwell plate is such that while the solution containing both the enzyme and a particular substrate candidate is sipped on one sipper of a sipper chip, the control solution containing that same substrate candidate is also sipped onto the same chip through another sipper. After the enzyme-containing and control solutions enter the microfluidic device, both solutions are subjected to a common set of separation conditions (i.e. pressure and voltages). The use of the control solution becomes most critical when there is a high level of substrate conversion in the reaction well, as only one peak is present and so comparison of arrival time of peaks in the channels containing the enzyme-containing and control solutions indicates the presence of product formation.

In general, to achieve a desired resolution during the separation of a given peptide substrate and the product resulting from the interaction between the substrate and an enzyme, an optimum set of pressures and separation voltages are used. This would mean that for a panel of 54 peptides, 54 unique separation conditions would be used. Even with the automation afforded by the use of a microfluidic device, it may be cumbersome to manually input the separation conditions required for each peptide. A compromise solution involves grouping peptides that have similar separation conditions and separating them under a common set of conditions. For example, a constant pressure could be applied during the assay of each peptide, while different voltages could be applied for each different group of peptides.

In many embodiments, the substrate-candidate peptides can be broadly grouped into two groups: product-first and substrate-first assays. In a product-first assay, the product peak of a product-substrate pair reaches the detector first. An operating pressure was designated for product- and substrate-first assays; in our specific case we chose -1 psi for product-first assays and -1.6 for substrate-first assays. Next, voltage drops were chosen that give a resolution of 1.2 at the operating pressure. Peptides were then grouped together into groups of 6 where the operating voltage drops were within 200V of each other. Peptides were grouped into groups of 6 because a 12-sipper chip could sip 6 enzyme-containing wells and 6 control wells simultaneously. (Note: if a 4 sipper chip were used, peptides could be grouped into groups of 2.) An operating voltage drop was then chosen for this group of 6 peptides by choosing highest voltage drop common to all 6 peptides; in this way each product-substrate pair had a resolution of 1.2 or greater and assay sensitivity was not compromised. In some cases a group of 6 peptides could not be made and so a group of fewer than six was made with the balance being buffer-only wells. So in the case of 54 peptides used in our library, 21 were chosen to be product-first, which translated into 4 groups of 6 peptides with 3 buffer only wells. All groups operated at the same pressure of -1 psi, but each group had an incrementally increasing voltage drop as designated within a multi-line voltage script. The timing of application of the successive voltage drops was determined such that the peptides from the previous group had passed the detection zone and the new peptides had not yet been sipped. This in turn, coupled with the separation conditions, determined the throughput of the assay. The remaining 33 peptides were grouped on a separate plate and run substrate-first at a common pressure of -1.6 psi, resulting in 6 groups of 6 peptides with 3 buffer-only wells. Choice of voltage and timing of application of voltage is identical to the product-first case.

The substrates are placed on a multiwell plate in DMSO in a concentrated format such that when the enzyme and reaction buffer is used, the final substrate concentration is close to the desired assay conditions, e.g., store 1 µl of 76 gM substrate and then add to 50 p1 of reaction buffer for a final concentration of 1.5 µM. These plates can then be made in bulk and frozen for use at a later date.

Without knowledge of the enzymatic kinetics, the reactions are typically carried out with a multi-hour incubation (> 12 hours) and with high enzyme and ATP concentrations.

With this assay format, 12 kinase enzymes were run to look for substrates. One enzyme was PICA and was used only as a test case to see if the method found our regular kinase target.

Of the other 11 kinases, we found new targets for 8 of them. These enzymes and their new substrates are listed in the following table.

| **Enzyme** | **[E] (nM)** | **[ATP] (µM)** | **CoFactor** | **Literature Substrate (% Conversion)** | **Hits** |
|---|---|---|---|---|---|
| MET | 10 | 250 | MnCl₂ | KKKSPGEYVNIEFG (70%) | FL-EDPIYEFLPAKKK-CONH2 (97%) |
| | | | | | FL-MAEEEIYGEFFAKKK-CONH2 (93%) |
| | | | | | FL-KMAEEEEYFELVAKKK-CONH2 (90%) |
| | | | | | FL-EAIYAAPFAKKK-CONH2 (88%) |
| | | | | | FL-EEEEYFFIIAKKK-CONH2 (86%) |
| | | | | | FL-KMAEEEEYVFIEAKKK-CONH2 (85%) |
| | | | | | FL-KEDPDYEWPSAK-CONH2(63%) |
| | | | | | FL-MAAEEEYFFLFAKKK-CONH2 (62%) |
| | | | | | FL-EGIYGVLFKKK-CONH2 (60%) |
| MST2 | 10 | 100 | MnCl₂ | MBP (N/A) | FL-KKSRGDYMTMQIG-CONH2 (75%) |
| | | | | | FL-ENDYINASLKKK-CONH2 (60%) |
| | | | | | FL-PLARTLSVAGLPGKK-COOH(40%) |
| IKKα | 3.5 | 100 | MnCl₂ | IKK-tide (N/A) | FL-AKRRRLSSLRA-COOH (43%) |
| IGF-1R | 10 | 250 | MnCl₂ | KKKSPGEYVNIEFG (30%) | FL-KKSRGDYMTMQIG-CONH2 (55%) |
| | | | | | FL-MAAEEEYFFLFAKKK-CONH2 (60%) |
| FGFR3 | 5 | 250 | MnCl₂ | Poly-Glu Tyr (N/A) | FL-EDPIYEFLPAKKK-CONH2 (100%) |
| | | | | | FL-KMAEEEEYFELVAKKK-CONH2 (100%) |
| | | | | | FL-KMAEEEEYFELVAKKK-CONH2 (100%) |
| | | | | | FL-KMAEEEEYVFIEAKKK-CONH2 (100%) |
| | | | | | FL-MAAEEEYMMMMAKKK-CONH2 (78%) |
| | | | | | FL-KKKSPGEYVNIEFG-CONH2 (73%) |
| EGFR | 5 | 250 | MnCl₂ | Angiotensin II (N/A) | FL-EDPIYEFLPAKKK-CONH2 (98%) |
| | | | | | FL-KMAEEEEYFELVAKKK-CONH2(90%) |
| | | | | | FL-MAEEEIYGEFFAKKK-CONH2 (87%) |
| | | | | | FL-KKKSPGEYVNIEFG-CONH2 (50%) |
| | | | | | FL-KEDPDYEWPSAK-CONH2 (50%) |
| | | | | | FL-EAIYAAPFAKKK-CONH2 (46%) |
| | | | | | FL-ENDYINASLKKK-CONH2(44%) |
| IKKβ | | | MnCl₂ | IKK-tide (N/A) | FL-AKRRRLSSLRA-COOH (74%) |
| ERK2 (MAPK2) | | | | | FL-IPTSPITTTYFFFKKK-COOH (100%) |
| | | | | | FL-APRTPGGRR-COOH (60%) |
| MEK1 | 7 | 100 | MnCl₂ | N/A | No Hit |
| JNK2a | 60 | 100 | MnCl₂ | N/A | No Hit |
| MKK6 | | | | | No Hit |

This table lists the substrates as hits with the peptide sequence and the 90 conversion in parentheses. Where possible, data is shown for the enzyme with a known peptide sequence for the enzyme. In addition, assay conditions are listed. In all cases, the enzyme and substrate were incubated overnight (i.e., ∼ 12-16 hours).

## Claims

1. A kit for screening a compound for enzyme inhibition activity using a microfluidic device (110), the microfluidic device having at least one microchannel (102) and a capillary element (112), the capillary element operably connected to and in fluid communication with the microchannel, the kit comprising:
a first multiwell plate (150) having a plurality of enzymes disposed within a first plurality of wells;
a second multiwell plate (160) having a plurality of enzyme substrates disposed within a second plurality of wells, each one of the second plurality of wells corresponding to one of the first plurality of wells;
a phosphate source (162) disposed within each well of the second plate, the phosphate source to be disposed in each of the well at a predetermined phosphate source concentration;
a cofactor (164) disposed within each well of the second plate, the cofactor disposed in each well at a predetermined cofactor concentration;
wherein the enzyme within each well of the first plate is selected to react with the enzyme substrate, the phosphate source, and the cofactor in the corresponding well of the second plate when the compound is not present; and
an instruction set stored on a computer readable medium, the instruction set comprising instructions for controlling dipping of capillary elements into the first plurality of wells; **characterized in that** the microfluidic device has a first reservoir at one end of the microchannel and a second reservoir at an opposite terminus of the microchannel, the first reservoir having a depth greater than the depth of the second reservoir, the first reservoir having a larger volume relative to the volume or flow rate of the at least one microchannel such that a sample is flowable into the at least one microchannel of the microfluidic device via the capillary element of the microfluidic device by a hydrostatic pressure differential created by the depth differential between the first reservoir and the second reservoir.

2. The kit of claim 1 wherein the first plurality of wells of the first multiwell plate are disposed evenly upon the plate in 24 columns (250), each column configured for including a different enzyme disposed within the wells of the column.

3. The kit of claim 2 wherein the order in which the enzymes are disposed on the first plate is based on similarity of electrophoretic mobility.

4. The kit of claim 2 wherein the order in which the enzymes are disposed on the first plate from column 1 to column 24 is MAPKAPK2, AurA, PKCζ RSK1, MAPKAPK5, Erk1, PKD2, CK1δ, CHK1, ABL, FYN, LYNa, CHK2, MET, LCK, SRC, GSK3β, Erk2, PKACα, AKT2, INSR, p38a, AKT1, and MSK1.

5. The kit of claim 2 wherein the order in which the enzymes are disposed on the first plate from column 1 to column 24 is PKCβ2, ROCK2, CDK2, MST2, PKG1α, PAK2, IGF1R, FGFR1, MARK1, CAMK2δ, PIM2, BTK, c-TAK1, DYRK1a, CaMK4, AMPKal, FLT3, HGK, KDR, Raf-1, P70S6K, IRAK4, SGK, and SYK.

6. The kit of claim 1 further comprising a reconstitution buffer (152).

7. The kit of claim 1 further comprising a termination buffer (158).

8. The kit of claim 1 wherein the plurality of enzymes are sealed in the first plurality of wells, and the plurality of enzyme substrates, the phosphate source, and the cofactor are sealed in within the second plurality of wells.

9. The kit of claim 1 wherein at least some of the first plurality of wells are control wells.

10. A system for screening a compound for enzyme inhibition activity, the system comprising:
a kit including a first multiwell plate (150) and a second multiwell plate (160),
the first multiwell plate having a plurality of enzymes disposed within a first plurality of wells,
the second multiwell plate having a plurality of enzyme substrates disposed within a second plurality of wells, each one of the second plurality of wells corresponding to one of the first plurality of wells ,
a phosphate source (162) disposed at a predetermined phosphate source concentration within each well of the second multiwell plate,
a cofactor (164) disposed at a predetermined cofactor concentration within each well of the second multiwell plate, and wherein the enzyme for being disposed within each well of the first plate is selected to react with the enzyme substrate, the phosphate source, and the cofactor in the corresponding well of the second plate when the compound is not present;
a microfluidic device (110), the microfluidic device having at least one microchannel (102) and a capillary element (112), the capillary element operably connected to and in fluid communication with the microchannel; and
an instruction set stored on a computer readable medium, the instruction set comprising instructions for controlling dipping of the capillary element into a respective well of the first plurality of wells; **characterized in that** the microfluidic device has a first reservoir at one end of the microchannel and a second reservoir at an opposite terminus of the microchannel, the first reservoir having a depth greater than the depth of the second reservoir, the first reservoir having a large volume relative to the volume or flow rate of the at least one microchannel, such that a sample is flowable into the at least one microchannel of the microfluidic device via the capillary element of the microfluidic device by a hydrostatic pressure differential created by the depth differential between the first reservoir and the second reservoir.

11. The system of claim 10 further comprising a detector (122) operably connected to the microfluidic device and a computer (120).

12. The system of claim 11 further comprising a controller (130) operably connected to the computer 120.

13. The system of claim 12 further comprising a fluid direction system (140) operably connected to the computer (120), the fluid direction system including a pressure source (142) in fluid communication with the microfluidic device.

14. The system of claim 10 wherein the plurality of enzymes are sealed in the first plurality of wells, and the plurality of enzyme substrates, the phosphate source, and the cofactor are sealed in the second plurality of wells.

15. The system of claim 10 wherein at least some of the first plurality of wells are control wells.

## Patentansprüche

1. Set zum Screenen einer Verbindung auf Enzymhemmungsaktivität unter Verwendung einer Mikrofluidvorrichtung (110), wobei die Mikrofluidvorrichtung zumindest einen Mikrokanal (102) und ein Kapillarelement (112) aufweist, wobei das Kapillarelement mit dem Mikrokanal operabel verbunden ist und mit diesem in Fluidkommunikation steht, wobei das Set Folgendes umfasst:
eine erste Multiwellplatte (150) mit einer Vielzahl von Enzymen, die in einer ersten Vielzahl von Wells angeordnet sind;
eine zweite Multiwellplatte (160) mit einer Vielzahl von Enzymsubstraten, die in einer zweiten Vielzahl von Wells angeordnet sind, wobei jedes aus der zweiten Vielzahl von Wells einem aus der ersten Vielzahl von Wells entspricht;
eine Phosphatquelle (162), die in jedem Well der zweiten Platte angeordnet ist, wobei die Phosphatquelle in jedem Well in einer vorbestimmten Phosphatquellenkonzentration angeordnet ist;
einen Cofaktor (164), der in jedem Well der zweiten Platte angeordnet ist, wobei der Cofaktor in jedem Well in einer vorbestimmten Cofaktorkonzentration angeordnet ist;
wobei das Enzym in jedem Well der ersten Platte ausgewählt ist, um mit dem Enzymsubstrat, der Phosphatquelle und dem Cofaktor in dem entsprechenden Well der zweiten Platte zu reagieren, wenn die Verbindung nicht vorhanden ist; und
einen Befehlssatz, der auf einem computerlesbaren Medium gespeichert ist, wobei der Befehlssatz Befehle zur Steuerung des Eintauchens von Kapillarelementen in die erste Vielzahl von Wells umfasst;
**dadurch gekennzeichnet, dass** die Mikrofluidvorrichtung einen ersten Sammelbehälter an einem Ende des Mikrokanals und einen zweiten Sammelbehälter an einem entgegengesetzten Ende des Mikrokanals aufweist, wobei der erste Sammelbehälter eine größere Tiefe als die Tiefe des zweiten Sammelbehälters aufweist, der erste Sammelbehälter ein größeres Volumen im Vergleich zum Volumen oder der Durchflussrate des zumindest einen Mikrokanals aufweist, so dass eine Probe durch einen hydrostatischen Druckunterschied, der durch den Tiefenunterschied zwischen dem ersten Sammelbehälter und dem zweiten Sammelbehälter erzeugt wird, über die Kapillarelemente der Mikrofluidvorrichtung in den zumindest einen Mikrokanal der Mikrofluidvorrichtung fließen kann.

2. Set nach Anspruch 1, wobei die erste Vielzahl von Wells der ersten Multiwellplatte gleichmäßig auf der Platte in 24 Spalten (250) angeordnet ist, wobei jede Spalte ausgelegt ist, um ein anderes Enzym zu umfassen, das in den Wells der Spalte angeordnet ist.

3. Set nach Anspruch 2, wobei die Reihenfolge, in der die Enzyme auf der ersten Platte angeordnet sind, auf einer Ähnlichkeit der elektrophoretischen Mobilität basiert.

4. Set nach Anspruch 2, wobei die Reihenfolge, in der die Enzyme auf der ersten Platte von Spalte 1 zu Spalte 24 angeordnet sind, MAPKAPK2, AurA, PKCζRSK1, MAPKAPK5, Erk1, PKD2, CK1δ, CHK1, ABL, FYN, LYNa, CHK2, MET, LCK, SRC, GSK3β, Erk2, PKACα, AKT2, INSR, p38a, AKT1 und MSK1 ist.

5. Set nach Anspruch 2, wobei die Reihenfolge, in der die Enzyme auf der ersten Platte von Spalte 1 zu Spalte 24 angeordnet sind, PKCβ2, ROCK2, CDK2, MST2, PKG1α, PAK2, IGF1R, FGFR1, MARK1, CAMK2δ, PIM2, BTK, c-TAK1, DYRK1a, CaMK4, AMPKα1, FLT3, HGK, KDR, Raf-1, P70S6K, IRAK4, SGK und SYK ist.

6. Set nach Anspruch 1, das außerdem einen Rekonstitutionspuffer (152) umfasst.

7. Set nach Anspruch 1, das außerdem einen Terminationspuffer (158) umfasst.

8. Set nach Anspruch 1, wobei die Vielzahl von Enzymen in der ersten Vielzahl von Wells eingeschlossen ist und die Vielzahl von Enzymsubstraten, die Phosphatquelle und der Cofaktor in der zweiten Vielzahl von Wells eingeschlossen sind.

9. Set nach Anspruch 1, wobei zumindest manche aus der ersten Vielzahl von Wells Kontrollwells sind.

10. System zum Screenen einer Verbindung auf Enzymhemmungsaktivität, wobei das System Folgendes umfasst:
ein Set, umfassend eine erste Multiwellplatte (150) und eine zweite Multiwellplatte (160),
wobei die erste Multiwellplatte eine Vielzahl von Enzymen aufweist, die in einer ersten Vielzahl von Wells angeordnet sind;
die zweite Multiwellplatte eine Vielzahl von Enzymsubstraten aufweist, die in einer zweiten Vielzahl von Wells angeordnet sind, wobei jedes aus der zweiten Vielzahl von Wells einem aus der ersten Vielzahl von Wells entspricht;
eine Phosphatquelle (162), die in jedem Well der zweiten Multiwellplatte in einer vorbestimmten Phosphatquellenkonzentration angeordnet ist;
einen Cofaktor (164), der in jedem Well der zweiten Multiwellplatte in einer vorbestimmten Cofaktorkonzentration angeordnet ist, und wobei das Enzym, das in jedem Well der ersten Platte angeordnet werden soll, ausgewählt ist, um mit dem Enzymsubstrat, der Phosphatquelle und dem Cofaktor in dem entsprechenden Well der zweiten Platte zu reagieren, wenn die Verbindung nicht vorhanden ist;
eine Mikrofluidvorrichtung (110), wobei die Mikrofluidvorrichtung zumindest einen Mikrokanal (102) und ein Kapillarelement (112) aufweist, wobei das Kapillarelement mit dem Mikrokanal operabel verbunden ist und mit diesem in Fluidkommunikation steht; und
einen Befehlssatz, der auf einem computerlesbaren Medium gespeichert ist, wobei der Befehlssatz Befehle zur Steuerung des Eintauchens des Kapillarelements in ein entsprechendes Well der ersten Vielzahl von Wells umfasst;
**dadurch gekennzeichnet, dass** die Mikrofluidvorrichtung einen ersten Sammelbehälter an einem Ende des Mikrokanals und einen zweiten Sammelbehälter an einem entgegengesetzten Ende des Mikrokanals aufweist, wobei der erste Sammelbehälter eine größere Tiefe als die Tiefe des zweiten Sammelbehälters aufweist, der erste Sammelbehälter ein größeres Volumen im Vergleich zum Volumen oder der Durchflussrate des zumindest einen Mikrokanals aufweist, so dass eine Probe durch einen hydrostatischen Druckunterschied, der durch den Tiefenunterschied zwischen dem ersten Sammelbehälter und dem zweiten Sammelbehälter erzeugt wird, über die Kapillarelemente der Mikrofluidvorrichtung in den zumindest einen Mikrokanal der Mikrofluidvorrichtung fließen kann.

11. System nach Anspruch 10, das außerdem einen Detektor (122) umfasst, der mit der Mikrofluidvorrichtung und einem Computer (120) operabel verbunden ist.

12. System nach Anspruch 11, das außerdem eine Steuerung (130) umfasst, die mit dem Computer (120) operabel verbunden ist.

13. System nach Anspruch 12, das außerdem ein Fluidlenksystem (140) umfasst, das mit dem Computer (120) operabel verbunden ist, wobei das Fluidlenksystem eine Druckquelle (142) umfasst, die mit der Mikrofluidvorrichtung in Fluidkommunikation steht.

14. System nach Anspruch 10, wobei die Vielzahl von Enzymen in der ersten Vielzahl von Wells eingeschlossen ist und die Vielzahl von Enzymsubstraten, die Phosphatquelle und der Cofaktor in der zweiten Vielzahl von Wells eingeschlossen sind.

15. System nach Anspruch 10, wobei zumindest manche aus der ersten Vielzahl von Wells Kontrollwells sind.

## Revendications

1. Kit pour cribler un composé pour une activité d'inhibition d'enzymes en utilisant un dispositif microfluidique (110), le dispositif microfluidique comportant au moins un microcanal (102) et un élément capillaire (112), l'élément capillaire étant en connexion fonctionnelle et en communication fluidique avec le microcanal, le kit comprenant :
une première plaque multipuits (150) comportant une pluralité d'enzymes disposées dans une première pluralité de puits ;
une seconde plaque multipuits (160) comportant une pluralité de substrats enzymatiques disposés dans une seconde pluralité de puits, chacun de la seconde pluralité de puits correspondant à l'un de la première pluralité de puits ;
une source de phosphate (162) disposée dans chaque puits de la seconde plaque, la source de phosphate étant disposée dans chacun des puits à une concentration de source de phosphate prédéterminée ;
un cofacteur (164) disposé dans chaque puits de la seconde plaque, le cofacteur étant disposé dans chaque puits à une concentration de cofacteur prédéterminée ;
dans lequel l'enzyme dans chaque puits de la première plaque est sélectionnée pour réagir avec le substrat enzymatique, la source de phosphate, et le cofacteur dans le puits correspondant de la seconde plaque lorsque le composé n'est pas présent ; et
un ensemble d'instructions stocké sur un support lisible par ordinateur, l'ensemble d'instructions comprenant des instructions pour contrôler l'immersion des éléments capillaires dans la première pluralité de puits ;
**caractérisé en ce que** le dispositif microfluidique possède un premier réservoir à une extrémité du microcanal et un second réservoir à une extrémité opposée du microcanal, le premier réservoir possédant une profondeur plus importante que la profondeur du second réservoir, le premier réservoir possédant un volume plus important par rapport au volume ou au débit du au moins un microcanal de sorte qu'un échantillon peut circuler dans le au moins un microcanal du dispositif microfluidique via l'élément capillaire du dispositif microfluidique par un différentiel de pression hydrostatique créé par le différentiel de profondeur entre le premier réservoir et le second réservoir.

2. Kit selon la revendication 1, dans lequel la première pluralité de puits de la première plaque multipuits est disposée de manière uniforme sur la plaque en 24 colonnes (250), chaque colonne étant configurée pour inclure une enzyme différente disposée dans les puits de la colonne.

3. Kit selon la revendication 2, dans lequel l'ordre dans lequel les enzymes sont disposées sur la première plaque est basé sur la similarité de la mobilité électrophorétique.

4. Kit selon la revendication 2, dans lequel l'ordre dans lequel les enzymes sont disposées sur la première plaque de la colonne 1 à la colonne 24 est MAPKAPK2, AurA, PKCζP3K1, MAPKAPK5, Erkl, PKD2, CK1δ, CHK1, ABL, FYN, LYNa, CHK2, MET, LCK, SRC, GSK3β, Erk2, PKACα, AKT2, INSR, p38α, AKT1, et MSK1.

5. Kit selon la revendication 2, dans lequel l'ordre dans lequel les enzymes sont disposées sur la première plaque de la colonne 1 à la colonne 24 est PKCβ2, ROCK2, CDK2, MST2, PKG1α, PAK2, IGF1R, FGFR1, MARK1, CAMK2δ, PIM2, BTK, c-TAK1, DYRK1a, CaMK4, AMPKα1, FLT3, HGK, KDR, Raf-1, P70S6K, IRAK4, SGK, et SYK.

6. Kit selon la revendication 1, comprenant en outre un tampon de reconstitution (152).

7. Kit selon la revendication 1, comprenant en outre un tampon de terminaison (158).

8. Kit selon la revendication 1, dans lequel la pluralité d'enzymes est scellée dans la première pluralité de puits, et la pluralité de substrats enzymatiques, la source de phosphate, et le cofacteur sont scellés dans la seconde pluralité de puits.

9. Kit selon la revendication 1, dans lequel au moins certains puits de la première pluralité de puits sont des puits de contrôle.

10. Système pour cribler un composé pour une activité d'inhibition d'enzymes, le système comprenant :
un kit comprenant une première plaque multipuits (150) et une seconde plaque multipuits (160),
la première plaque multipuits comportant une pluralité d'enzymes disposées dans une première pluralité de puits,
la seconde plaque multipuits comportant une pluralité de substrats enzymatiques disposés dans une seconde pluralité de puits, chacun de la seconde pluralité de puits correspondant à l'un de la première pluralité de puits,
une source de phosphate (162) disposée à une concentration de source de phosphate prédéterminée dans chaque puits de la seconde plaque multipuits,
un cofacteur (164) disposé à une concentration de cofacteur prédéterminée dans chaque puits de la seconde plaque multipuits, et dans lequel l'enzyme qui est disposée dans chaque puits de la première plaque est sélectionnée pour réagir avec le substrat enzymatique, la source de phosphate, et le cofacteur dans le puits correspondant de la seconde plaque lorsque le composé n'est pas présent ;
un dispositif microfluidique (110), le dispositif microfluidique comportant au moins un microcanal (102) et un élément capillaire (112), l'élément capillaire étant en connexion fonctionnelle et en communication fluidique avec le microcanal ; et
un ensemble d'instructions stocké sur un support lisible par ordinateur, l'ensemble d'instructions comprenant des instructions pour contrôler l'immersion de l'élément capillaire dans un puits respectif de la première pluralité de puits ;
**caractérisé en ce que** le dispositif microfluidique possède un premier réservoir à une extrémité du microcanal et un second réservoir à une extrémité opposée du microcanal, le premier réservoir possédant une profondeur plus importante que la profondeur du second réservoir, le premier réservoir possédant un volume important par rapport au volume ou au débit du au moins un microcanal, de sorte qu'un échantillon peut circuler dans le au moins un microcanal du dispositif microfluidique via l'élément capillaire du dispositif microfluidique par un différentiel de pression hydrostatique créé par le différentiel de profondeur entre le premier réservoir et le second réservoir.

11. Système selon la revendication 10, comprenant en outre un détecteur (122) en connexion fonctionnelle avec le dispositif microfluidique et un ordinateur (120).

12. Système selon la revendication 11, comprenant en outre un contrôleur (130) en connexion fonctionnelle avec l'ordinateur 120.

13. Système selon la revendication 12, comprenant en outre un système d'orientation de fluide (140) en connexion fonctionnelle avec l'ordinateur (120), le système d'orientation de fluide comprenant une source de pression (142) en communication fluidique avec le dispositif microfluidique.

14. Système selon la revendication 10, dans lequel la pluralité d'enzymes est scellée dans la première pluralité de puits, et la pluralité de substrats enzymatiques, la source de phosphate, et le cofacteur sont scellés dans la seconde pluralité de puits.

15. Système selon la revendication 10, dans lequel au moins certains puits de la première pluralité de puits sont des puits de contrôle.
